# EUROPEAN PATENT APPLICATION

(11) **EP 2 491 921 A1**
(43) Date of publication of application: **29.08.2012**
(21) Application number: 11155917.5
(22) Date of filing: 25.02.2011
(51) Int. Cl.: A61K 9/16, A61K 9/20, A61K 31/4353

(54) **Conservation of anhydrous form of gemifloxacin**

(71) Applicant: Deva Holding Anonim Sirketi, 34303 Istanbul (TR)
(72) Inventor: HAAS, Philipp Daniel, 34303, ISTANBUL (TR); KOC, Fikret, 34303, ISTANBUL (TR); SIVASLIGIL, Ramazan, 34303, ISTANBUL (TR); KANDEMIR, Levent, 34303, ISTANBUL (TR)

(57) **Abstract**

The invention encompasses wet granulation with an organic solvent, direct compression, dry granulation manufacturing methods and pharmaceutical formulations of anhydrous gemifloxacin mesylate.

## Description

### Technical Field

This invention encompasses a wet granulation with an organic solvent and a direct compression and a dry granulation manufacturing methods and pharmaceutical formulations to conserve anhydrous form of gemifloxacin.

### Background Art

Gemifloxacin is a synthetic broad-spectrum antibacterial agent that belongs to a class of antibiotics called fluoroquinolones.

Gemifloxacin was found to be especially effective against respiratory tract infections such as acute exacerbation of chronic bronchitis (AECB), community acquired pneumonia (CAP) and acute bacterial sinusitis (ABS).

Gemifloxacin was firstly disclosed by EP 688772 B (LG CHEMICAL LTD.) 05.06.1999 .EP688772 describes a novel quinoline carboxylic acid derivative having an antibacterial activity. The patent '772 discloses process for the preparation of gemifloxacin mesylate anhydrous, monohydrate, sesquihydrate and trihydrate.

As a methanesulfonate form, gemifloxacin also has good physicochemical properties such as high water solubility and stability.

Gemifloxacin is designated chemically as 7-[(4*Z*)-3-(aminomethyl) - 4-methoxyimino-pyrrolidin-1-yl] -1-cyclopropyl-6-fluoro-4-oxo- 1, 8-naphthyridine-3-carboxylic acid.

Published EP0981527 B (LG LIFE SCIENCES LTD.) 01.03.2000 discloses hydrate forms of gemifloxacin mesylate, especially sesquihydrate.

Gemifloxacin is available in an oral formulation as the mesylate salt in the sesquihydrate form and it is marketed under the name of "Factive".

### Summary of invention

This invention is directed to wet granulation with an organic solvent, direct compression, dry granulation manufacturing methods and pharmaceutical formulations to conserve anhydrous form of gemifloxacin mesylate.

### Description of embodiments

In the first aspect of this invention, a pharmaceutical formulation and manufacturing method of wet granulation with an organic solvent or mixtures of organic solvents for the preparation oral solid dosage form of anhydrous gemifloxacin mesylate is provided. It is invented that using of an organic solvent or mixture of organic solvents in wet granulation supply that anhydrous form of gemifloxacin is maintained during and after granulation. In storage, anhydrous property of gemifloxacin mesylate is kept due to using of organic solvents in wet granulation.

Hydrated forms of gemifloxacin mesylate tend more to be degraded than anhydrous form of gemifloxacin mesylate.

A preferred embodiment is created by careful handling of organic solvent granulation of anhydrous gemifloxacin mesylate. Organic solvent may be in a mixture with water wherein water is in an amount of non-convertible for anhydrous form of gemifloxacin.

In the present invention, inventors have found that anhydrous form of active ingredient is preserved without any change in crystalline structure with mentioned manufacturing methods. The anhydrous gemifloxacin mesylate is not transformed into any hydrated form of gemifloxacin mesylate.

In the present invention, the careful control of environmental factors is essential to the manufacturing of tablets of anhydrous gemifloxacin mesylate.

All manufacturing steps including granulation, blending, tabletting, coating are performed in controlled areas.

Additionally, fluid bed granulator can optionally have a dehumidification system in order to reduce humidity of fluidized inlet air.

In accordance with this invention, wet granulation with an organic solvent formulation comprises; anhydrous gemifloxacin mesylate is in the range of from about 10 % to about 90 %, diluent is in the range of from about 1 % to about 50 %, binder is in the range of from about 1 % to about 10%, disintegrant is in the range of from about 3 % to about 15 %, lubricant is in the range of from about 0.1% to about 2 % by weight of pharmaceutical dosage form.

Preferably wet granulation with an organic solvent formulation comprises; anhydrous gemifloxacin mesylate is in the range of from about 15 % to about 85 %, diluent is in the range of from about 2 % to about 40 %, binder is in the range of from about 2 % to about 6 %, disintegrant is in the range of from about 5 % to about 10 %, lubricant is in the range of from about 0.3 % to about 1.5 % by weight of pharmaceutical dosage form.

Most preferably, anhydrous gemifloxacin mesylate pharmaceutical composition comprising; gemifloxacin mesylate is 61.38% , diluent is 25.78 %, binder is 3.38 %, disintegrant is 8.46 %, lubricant is 1.00% by weight of total pharmaceutical dosage form.

For the present invention, in wet granulation with an organic solvent of anhydrous gemifloxacin mesylate, anhydrous gemifloxacin mesylate:
diluent ratio is from 1:7 to 7:1 by weight. More preferably ratio is from 1:6 to 6:1 and most preferably from 1:5 to 5:1 by weight.

Precisely, in wet granulation of anhydrous gemifloxacin mesylate with an organic solvent, anhydrous gemifloxacin mesylate: diluent ratio is 2.38.

For the present invention, in wet granulation of anhydrous gemifloxacin mesylate with an organic solvent, anhydrous gemifloxacin mesylate:
binder ratio is from 1:70 to 70:1 by weight. More preferably ratio is from 1:60 to 60:1 and most preferably ratio is selected from 1:30 to 30: 1.

Precisely, in wet granulation of anhydrous gemifloxacin mesylate with an organic solvent, anhydrous gemifloxacin mesylate: binder ratio is 18.16.

For the present invention, in wet granulation of anhydrous gemifloxacin mesylate with an organic solvent, anhydrous gemifloxacin mesylate :
disintegrant ratio is selected from 1:50 to 50:1 by weight. More preferably ratio is selected from 1:20 to 20:1 and most preferably from1:10 to 10:1.

Precisely, in wet granulation of anhydrous gemifloxacin mesylate with an organic solvent, anhydrous gemifloxacin mesylate: disintegrant ratio range is 7.25.

For the present invention, in wet granulation of anhydrous gemifloxacin mesylate with an organic solvent, anhydrous gemifloxacin mesylate:
lubricant ratio is selected from 1:200 to 200:1 by weight. More preferably ratio is selected from 1:100 to 100:1 and most preferably from 1:75 to 75:1.

Precisely, in wet granulation of anhydrous gemifloxacin mesylate with an organic solvent, anhydrous gemifloxacin mesylate: lubricant ratio is 61.38.

Organic solvents, used in preparation of solution are ,but not limited to, alcohols such as methanol, ethanol, propyl alcohol, isopropyl alcohol, 2-methyl-1-propanol ;ketones such as acetone, methyl ethyl ketone, methyl isobutyl ketone etc. ; esters such as, methyl acetate, ethyl acetate, propyl acetate, isopropyl acetate, ethyl formate, hydrocarbons such as heptane ; halogenated hydrocarbons such as dichloromethane and various other solvents such as acetonitrile, methylene chloride, toluene, and 1,1,1-trichloroethane. Mixtures of an organic solvent such as methanol, ethanol or acetone are particularly preferable.

In addition, the solvent is volatile, non toxic and removable to a level that is acceptable according to The International Committee on Harmonization (ICH) guidelines by drying.

The wet granulation with an organic solvent of the present invention is produced using a method for producing a preparation of granules, or fine granules, as exemplified by mixing-granulating method, extrusion-granulating method, fluidized bed-granulating method, chopping granulating method, spray-granulating method, and crushing granulating method, one-pot processing method.

Fluid bed granulator may optionally have dehumidification system in order to reduce humidity of fluidized inlet air. Dehumidification may be achieved by utilizing available techniques, such as, but not limited to, silica gel dehumidification or chilled water dehumidification.

In another aspect of this invention, dry manufacturing methods which do not involve any water contact. Dry manufacturing embraces direct compression and dry granulation methods.

Since water is not used during dry manufacturing, solution or solution mediated crystalline structure transformations are eliminated. With a water-free process, water induced degradation is avoided as well. Therefore, stability of finished pharmaceutical dosage form is enhanced and longer shelf lives are achievable.

In another preferred embodiment, direct compression manufacturing method is presented.

In accordance with this invention, direct compression formulation comprises; anhydrous gemifloxacin mesylate is in the range of from about 10 % to about 90 %, diluent is in the range of from about 1 % to about 50 %, binder is in the range of from about 1 % to about 10%, disintegrant is in the range of from about 3 % to about 15 %, lubricant is in the range of from about 0.1 % to about 2 % by weight of pharmaceutical dosage form.

Preferably direct compression formulation comprises; anhydrous gemifloxacin mesylate is in the range of from about 15 % to about 85%, diluent is in the range of from about 2 % to about 45 %, binder is in the range of from about 2 % to about 6 %, disintegrant is in the range of from about 5 % to about 10 %, lubricant is in the range of from about 0.3 % to about 1.5 % by weight of pharmaceutical dosage form.

Most preferably, anhydrous gemifloxacin mesylate pharmaceutical composition comprising; gemifloxacin mesylate is 46.94 %, diluent is 39.71 %, binder is 3.53 %, disintegrant is 8.82 %, lubricant is 1.00% by weight of total pharmaceutical dosage form.

For the present invention, in direct compression of anhydrous gemifloxacin mesylate, anhydrous gemifloxacin mesylate: diluent ratio is selected from 1:8 to 8:1 by weight. More preferably ratio is selected from 1:6 to 2:1 and most preferably from 1:1 to 2:1.

Precisely, in direct compression formulation of anhydrous gemifloxacin mesylate, anhydrous gemifloxacin mesylate: diluent ratio is 1.18.

For the present invention, in direct compression of anhydrous gemifloxacin mesylate, anhydrous gemifloxacin mesylate: binder ratio is selected from 1:80 to 80:1 by weight. More preferably ratio is selected from 1:50 to 50:1 and most preferably from 1:20 to 20:1.

Precisely, in direct compression formulation of anhydrous gemifloxacin mesylate, anhydrous gemifloxacin mesylate: binder ratio is 13.30.

For the present invention, in direct compression of anhydrous gemifloxacin mesylate, anhydrous gemifloxacin mesylate: disintegrant ratio is selected from 1:30 to 30:1 by weight. More preferably ratio is selected from 1:20 to 20:1 and most preferably from 1:10 to 10:1.

Precisely, in direct compression formulation of anhydrous gemifloxacin mesylate, anhydrous gemifloxacin mesylate: disintegrant ratio is 5.32.

For the present invention, in direct compression formulation of anhydrous gemifloxacin mesylate, anhydrous gemifloxacin mesylate: lubricant ratio is selected from 1:200 to 200:1 by weight. More preferably ratio is selected from 1:100 to 100:1 1 and most preferably from 1:50 to 50:1.

Precisely, in direct compression formulation of anhydrous gemifloxacin mesylate, anhydrous gemifloxacin mesylate: lubricant ratio is 46.94.

In another preferred embodiment, dry granulation manufacturing method which involves the use of forming compacts/slugs is presented.

In accordance with this invention, dry granulation formulation comprises; anhydrous gemifloxacin mesylate is in the range of from about 10 % to about 90 %, diluent is in the range of from about 1 % to about 50 %, binder is in the range of from about 1 % to about 10%, disintegrant is in the range of from about 3 % to about 15 %, lubricant is in the range of from about 0.1 % to about 2 % by weight of pharmaceutical dosage form.

Preferably dry granulation formulation comprises; anhydrous gemifloxacin mesylate is in the range of from about 15 % to about 85 %, diluent is in the range of from about 2 % to about 25 %, binder is in the range of from about 0.5 % to about 3.5 %, disintegrant is in the range of from about 4 % to about 8 %, lubricant is in the range of from about 0.3 % to about 1.5 % by weight of pharmaceutical dosage form.

Most preferably, anhydrous gemifloxacin mesylate pharmaceutical composition comprising anhydrous gemifloxacin mesylate is 79.80 %, diluent is 11.20%, binder is 2 %, disintegrant is 6 %, lubricant is 1 % by weight of total pharmaceutical dosage form.

For the present invention, in dry granulation formulation of anhydrous gemifloxacin mesylate, anhydrous gemifloxacin mesylate: diluent ratio is selected from 1:50 to 50:1 by weight. More preferably ratio is selected from 1:20 to 20:1 and most preferably from1:10 to 10:1.

Precisely, in dry granulation formulation of anhydrous gemifloxacin mesylate, anhydrous gemifloxacin mesylate: diluent ratio is 7.12.

For the present invention, in dry granulation formulation of anhydrous gemifloxacin mesylate, anhydrous gemifloxacin mesylate: binder ratio is selected from 1:200 to 200:1 by weight. More preferably ratio is selected from 1:100 to 100:1 and most preferably from 1:50 to 50:1.

Precisely, in dry granulation formulation of anhydrous gemifloxacin mesylate, anhydrous gemifloxacin mesylate: binder ratio is 39.90.

For the present invention, in dry granulation formulation of anhydrous gemifloxacin mesylate, anhydrous gemifloxacin mesylate: disintegrant ratio is selected from 1:80 to 80:1 by weight. More preferably ratio is selected from 1:50 to 50:1 and most preferably from 1:20 to 20: 1.

Precisely, in dry granulation formulation of anhydrous gemifloxacin mesylate, anhydrous gemifloxacin mesylate : disintegrant ratio is 13.30.

For the present invention of dry granulation formulation of anhydrous gemifloxacin mesylate, anhydrous gemifloxacin mesylate: lubricant ratio is selected from 1:200 to 200:1 by weight. More preferably ratio is selected from 1:150 to 150:1 and from 1:100 to 100:1.

Precisely, in dry granulation formulation of anhydrous gemifloxacin mesylate, anhydrous gemifloxacin mesylate: lubricant ratio is 79.80.

Formulations in accordance with the present invention include pharmaceutically acceptable diluents, binders, disintegrants and lubricants.

Diluents are inert bulking agents which are added to active pharmaceutical ingredient to make a reasonably sized tablet. Diluents are, but not limited to, anhydrous lactose, lactose monohydrate, modified lactose, dibasic calcium phosphate, tribasic calcium phosphate, microcrystalline cellulose, silicified microcrystalline cellulose, powdered cellulose, maize starch, pregelatinized starch, calcium carbonate, sucrose, glucose, dextrates, dextrins, dextrose, fructose, kaolin, lactitol, maltose, mannitol, sorbitol, starch, mixtures thereof and the like.

Binders are agents used to impart cohesive qualities to the powdered material. Binders impart a cohesiveness to the tablet formulation that ensures that the tablet remain intact after compression. Tablet binders used in the mixture include tablet binders commonly used for tablet preparation. Binders are, but not limited to, polyvinylpyrolidone, copovidone, starches such as pregelatinized starch or plain starch, cellulose derivatives such as hydroxypropylmethylcellulose, ethylcellulose, hydroxypropylcellulose and carboxymethylcellulose and their salts, gelatine, acacia, agar, alginic acid, carbomer, ceratonia, chitosan, dextrates, dextrin, glycerol dibehenate, guar gum, hypromellose, inulin, magnesium aluminumsilicate, maltodextrin, poloxamer, polycarbophil, polydextrose, polyethylene oxide, polymethacrylates, sodium alginate, sucrose, hydrogenated vegetable oil, mixtures thereof and the like.

Disintegrants are, but not limited to, modified starches, croscarmallose sodium, carboxymethylcellulose calcium, sodium starch glycolate, crospovidone, alginic acid, calcium alginate, microcrystalline cellulose, powdered cellulose, chitosan, colloidal silicon dioxide, crospovidone, guar gum, low-substituted hydroxypropylcellulose, hydroxypropyl starch, magnesium aluminium silicate, methylcellulose, polacrilin potassium, sodium alginate, starch, dried starch, pregelatinized starch, mixtures thereof and the like.

Lubricants prevent adhesion of the tablet material to equipment, reduce interparticle friction, and facilitate the ejection of the tablet from the die cavity. Tablet lubricants added to the milled dried granulate include those typically used in tablet formulations. Lubricants are, but not limited to, magnesium stearate, calcium stearate, hydrogenated castor oil, glyceryl behenate, glyceryl monostearate, glyceryl palmitostearate, leucine, mineral oil, light mineral oil, myristic acid, palmitic acid, polyethylene glycol, potassium benzoate, sodium benzoate, sodium lauryl sulfate, sodium stearyl fumarate, stearic acid, talc, hydrogenated vegetable oil, zinc stearate, magnesium lauryl sulphate, sodium stearyl fumarate, polyethylene glycol, stearic acid, colloidal silicon dioxide or mixtures thereof and the like.

The compositions of the present invention preferably also may contain a glidant. Glidants are preferably selected from the group consisting of colloidal silicon dioxide, precipitated silica and pyrogenic silica, talc and aluminium silicate, tribasic calcium phosphate, calcium stearate, powdered cellulose, magnesium oxide, magnesium silicate, magnesium trisilicate, starch, talc, mixtures thereof and the like.

Pharmaceutically acceptable additives other than those described above may be included in formulations of the present invention of anhydrous gemifloxacin mesylate.

The compositions may include flavour enhancers, preservatives, antiadherants, flavourings, colouring agents, surfactants, solubilizers, wetting agents and other excipients of common use. Additives will vary according to the type of compositions being manufactured and can be included in the pharmaceutical composition in any amount.

In another aspect of this invention, core tablets of anhydrous gemifloxacin mesylate can be optionally film coated.

In coating solution's preparation, class 2 or 3 solvents listed in ICH Harmonised Tripartite Guideline are suitable to use. In the present invention, film coating dispersion is prepared either with alcohol which is substantially free of water or mixtures of different organic solvents. Content of residual solvents are determined in final product following to ICH Harmonised Tripartite Guideline for Impurities "Guideline for Residual Solvents, Q3C (R4), current Step 4 version dated February 2009".

Polymeric film coating materials, that can be used, are for instance, ethylcellulose, hydroxymethylcellulose, polyvinyl alcohol, polyethylene glycol, cellulose acetate phthalate, hydroxypropylmethylcellulose phthalate, polyvinyl acetate phthalate and Eudragit. Hydroxypropyl metylcellulose or polyvinyl alcohol which may be used as partially hydrolyzed form is preferred.

Coating may further contain additional excipients suitable for pharmaceutical purposes, for example, titanium oxide, talc, ferric oxide and the like. Coating material can be prepared either in-house preparation or can be used as pre-prepared ready-to-use mixtures from coating material suppliers, for instance Colorcon, Kerry or ISP. As a ready-to-use coating agent, Opadry ® White YS-1 R-7002 (Colorcon) can also be used.

In order to minimize interaction between the drug and the coating or the drug and the environmental conditions during manufacturing or storage, a sub coat or a seal coat can be used.

Total amount of film coating applied to core tablets can vary between 1-5 % (w/w) of the core tablet. Preferred applied film coating is 2-3 % (w/w) of the core tablet.

### Example 1: Formulation Prepared by Wet Granulation

**Table 1**

| **Formulation Prepared by Wet Granulation** | | |
|---|---|---|
| | **mg/tablet** | **%(w/w)** |
| **Ingredients** | | |
| Anhydrous Gemifloxacin Mesylate | 398.98 | 61.38 |
| Microcrystalline Cellulose | 132.52 | 20.40 |
| Lactose Monohydrate | 35.00 | 5.38 |
| Povidone | 22.00 | 3.38 |
| Crospovidone | 55.00 | 8.46 |
| Magnesium Stearate | 6.50 | 1.00 |
| Total Core Tablet Weight | 650.00 | 100.00 |

### Example 2: Manufacturing Method of Example 1

Wet granulation with organic solvent method provides a preparation method which has following steps :

**A. Intragranulation**

1. Anhydrous gemifloxacin mesylate, diluent (microcrystalline cellulose) and a portion of disintegrant (crospovidone) are mixed.

2. Binder (povidone) is added to alcohol and mixed until it is completely dissolved.

3. Powder mixture in step (1) is granulated with solution in step (2) using suitable granulator.

4. Wet granules are dried using fluid bed drier, until loss on drying is less than 2%.

**B. Extragranulation**

5. Dry granules are mixed with remaining part of disintegrant (crospovidone) and diluent (lactose monohydrate).

6. Lubricant (magnesium stearate) is added and mixed.

7. Core tablets are compressed with suitable punches having average target weight 650 mg/tablet.

8. 10% (w/w) dispersion of Opadry ® White YS-1 R-7002 is prepared in methylene chloride: isopropyl alcohol mixture.

9. Core tablets are film coated with dispersion prepared in step (8), using film coating machine, Vector Hi-Coater VHC-1355 Film Coating Machine, until desired film coating applied.

### Example 3: Formulation Prepared by Direct Compression

**Table 2**

| **Formulation Prepared by Direct Compression** | | |
|---|---|---|
| | **mg/tablet** | **%(w/w)** |
| **Ingredients** | | |
| Anhydrous Gemifloxacin Mesylate | 398.98 | 46.94 |
| Microcrystalline Cellulose | 85.00 | 10.00 |
| Lactose Monohydrate | 252.52 | 29.71 |
| Copovidone | 30.00 | 3.53 |
| Crospovidone | 75.00 | 8.82 |
| Magnesium Stearate | 8.50 | 1.00 |
| Total Core Tablet Weight | 850.00 | 100.00 |

### Example 4: Manufacturing Method of Example 3

Direct compression method provides a preparation method which has following steps :

1. Anhydrous gemifloxacin mesylate, diluents (microcrystalline cellulose, lactose monohydrate) and binder (copovidone), disintegrant (crospovidone) are mixed.

2. Optionally, powder mixture in step (1) is sieved through suitable sized sieve and mixed again.

3. Lubricant (magnesium stearate) is added and mixed.

4. Core tablets are compressed with suitable punches having average target weight 850 mg/tablet.

5. 12% (w/w) dispersion of Opadry ® White YS-1 R-7002 is prepared in alcohol.

6. Core tablets are film coated with dispersion prepared in step (5), using film coating machine, until desired film coating applied.

### Example 5: Formulation Prepared by Dry Granulation

**Table 3**

| **Formulation Prepared by Dry Granulation** | | |
|---|---|---|
| | **mg/capsule** | **% (w/w)** |
| **Ingredients** | | |
| Anhydrous Gemifloxacin Mesylate | 398.98 | 79.80 |
| Microcrystalline Cellulose | 56.02 | 11.20 |
| Copovidone | 10.00 | 2.00 |
| Crospovidone | 30.00 | 6.00 |
| Magnesium Stearate | 5.00 | 1.00 |
| Total Powder Weight in Capsule | 500.00 | 100.00 |

### Example 6: Manufacturing Method of Example 5

Dry granulation method provides a preparation method which has following steps :

1. Gemifloxacin mesylate anhydrous, diluent (microcrystalline cellulose), binder (copovidone) and a portion of disintegrant (crospovidone) are mixed.

2. Optionally, powder mixture in step (1) is sieved through suitable sized sieve and mixed again.

3. Powder mixture in step (2) is dry granulated with roller compactor or slugged with suitable punches.

4. Compacted granules or slug tablets are crushed and sieved through suitable sized sieve.

5. Granules in step (4) are mixed with the remaining of disintegrant (crospovidone).

6. Lubricant (magnesium stearate) is added and mixed.

7. Powder mixture in Step (6) is filled into gelatin capsules having average target powder weight of 500 mg/capsule.

## Claims

1. A wet granulation of anhydrous gemifloxacin or pharmaceutically acceptable salt thereof **characterized in that** wetting agent is organic solvent or mixture of organic solvents which are selected from group of methanol, ethanol, propyl alcohol, isopropyl alcohol, 2-methyl-1-propanol , acetone, methyl ethyl ketone, methyl isobutyl ketone, methyl acetate, ethyl acetate, propyl acetate, isopropyl acetate, ethyl formate, heptanes, dichloromethane ,acetonitrile, methylene chloride, toluene, and 1,1,1-trichloroethane.

2. The wet granulation with organic solvent, as claimed in claim 1, comprises following steps; a) anhydrous gemifloxacin mesylate, diluent and a portion of disintegrant are mixed and, b) binder is added to organic solvent and mixed until it is completely dissolved or dispersed and, c) powder mixture in step (a) is granulated with solution in step (b) using suitable granulator and, d) wet granules are dried using fluid bed drier, until target loss on drying achieved and, e) dry granules are mixed with remaining part of disintegrant and diluents and, f) lubricant is added and mixed and, g) core tablets are compressed with suitable punches and, h) a dispersion or solution of polymeric coating material which is prepared in single organic solvent or mixture or two or more and, i) core tablets are film coated with dispersion or solution prepared in step (h) until desired film coating applied.

3. Wet granulation, as claimed in claim 1, is used for manufacturing a pharmaceutical composition of anhydrous gemifloxacin mesylate which comprises: anhydrous gemifloxacin mesylate is in the range of from 10 % to 90 %, diluent is in the range of from 1 % to 50 %, binder is in the range of from 1 % to 10%, disintegrant is in the range of from 3 % to 15 %, lubricant is in the range of from 0.1 % to 2 % by weight of pharmaceutical dosage form:
**preferably,** anhydrous gemifloxacin mesylate is in the range of from 15 % to 85 %, diluent is in the range of from 2 % to 40 %, binder is in the range of from 2 % to 6 %, disintegrant is in the range of from 5 % to 10 %, lubricant is in the range of from 0.3 % to 1.5 % by weight of pharmaceutical dosage form: most **preferably,** anhydrous gemifloxacin mesylate is 61.38%, diluent is 25.78 %, binder is 3.38 %, disintegrant is 8.46 %, lubricant is 1.00% by weight of total pharmaceutical dosage form.

4. Wet granulation which is used for manufacturing a pharmaceutical composition of anhydrous gemifloxacin mesylate, as claimed in claim 1, comprises: **a)** anhydrous gemifloxacin mesylate: diluent ratio is from 1:7 to 7:1 by weight, **more preferably** ratio is from 1:6 to 6:1 and **most preferably** ratio is from1:5 to 5:1, **precisely** anhydrous gemifloxacin mesylate: diluent ratio is 2.38 **and/or,b)** anhydrous gemifloxacin mesylate: binder ratio is from 1:70 to 70:1 by weight, **more preferably** ratio is from 1:60 to 60:1 and **most preferably** ratio is from1:30 to 30:1, **precisely** anhydrous gemifloxacin mesylate: binder ratio is 18.16 **and/or,c)** anhydrous gemifloxacin mesylate: disintegrant ratio is from 1:50 to 50:1 by weight, **more preferably** ratio is from 1:20 to 20:1 and most preferably ratio is from1:10 to 10:1, **precisely** anhydrous gemifloxacin mesylate:
disintegrant ratio is 7.25 **and/or, d)** anhydrous gemifloxacin mesylate: lubricant ratio is from 1:200 to 200:1 by weight, **more preferably** ratio is from 1:100 to 100:1 and **most preferably** ratio is from1:75 to 75:1, **precisely** anhydrous gemifloxacin mesylate: lubricant ratio is 61.38.

5. A pharmaceutical composition comprises anhydrous gemifloxacin or pharmaceutically acceptable salt thereof and an excipient or mixtures of excipients **characterized in that** said composition is manufactured by direct compression method.

6. The direct compression, as claimed in claim 5, comprises following steps; a) anhydrous gemifloxacin mesylate, diluents, binder and disintegrant are mixed, b) optionally, powder mixture in step (a) is sieved through suitable sized sieve and mixed again, c) lubricant is added and mixed, d) core tablets are compressed with suitable punches, e) a dispersion or solution of polymeric coating material which is prepared in single organic solvent or mixture or two or more and, f) core tablets are film coated with dispersion or solution prepared in step (e) until desired film coating applied.

7. Direct compression, as claimed in claim 5, is used for manufacturing a pharmaceutical composition of anhydrous gemifloxacin mesylate comprises :
anhydrous gemifloxacin mesylate is in the range of from 10 % to 90 %, diluent is in the range of from 1 % to 50 %, binder is in the range of from 1 % to 10%, disintegrant is in the range of from 3 % to 15 %, lubricant is in the range of from 0.1 % to 2 % by weight of pharmaceutical dosage form, **morepreferably** anhydrous gemifloxacin mesylate is in the range of from 15 % to 85%, diluent is in the range of from 2 % to 45 %, binder is in the range of from 2 % to 6 %, disintegrant is in the range of from 5 % to 10 %, lubricant is in the range of from 0.3 % to 1.5 % by weight of pharmaceutical dosage form, **most preferably** anhydrous gemifloxacin mesylate is 46.94 %, diluent is 39.71 %, binder is 3.53 %, disintegrant is 8.82 %, lubricant is 1.00% by weight of total pharmaceutical dosage form.

8. The direct compression, as claimed in claim 5, is used for manufacturing a pharmaceutical composition of anhydrous gemifloxacin mesylate comprises: **a)** anhydrous gemifloxacin mesylate: diluent ratio is from 1:8 to 8:1 by weight, **more preferably** ratio is from 1:2 to 2:1, **most preferably** ratio is from 1:1 to 2:1, **precisely** anhydrous gemifloxacin mesylate: diluent ratio is 1.18 **and/or, b)** anhydrous gemifloxacin mesylate : binder ratio is from 1:80 to 80:1 by weight, **more preferably** ratio is from 1:50 to 50:1, **most preferably** ratio is from 1:20 to 20:1, **precisely** anhydrous gemifloxacin mesylate: binder ratio is 13.30 **and/or,** c) anhydrous gemifloxacin mesylate: disintegrant ratio is from 1:30 to 30:1 by weight, **more preferably** ratio is from 1:20 to 20:1, **most preferably** ratio is from 1:10 to 10:1, **precisely** anhydrous gemifloxacin mesylate: disintegrant ratio is 5.32 **and/or, d)** anhydrous gemifloxacin mesylate: lubricant ratio is from 1:200 to 200:1 by weight, **more preferably** ratio is from 1:100 to 100:1, **most preferably** ratio is from 1:50 to 50:1, **precisely** anhydrous gemifloxacin mesylate: lubricant ratio is 46.94.

9. A pharmaceutical composition comprises anhydrous gemifloxacin or pharmaceutically acceptable salt thereof and an excipient or mixtures of excipients **characterized in that** said composition is manufactured by dry granulation method.

10. The dry granulation, as claimed in claim 9, comprises following steps; a) anhydrous gemifloxacin mesylate, diluent, binder and a portion of disintegrant are mixed, b) optionally powder mixture in step (a) is sieved through suitable sized sieve and mixed again, c) powder mixture in step (b) is dry granulated with roller compactor or slugged with suitable punches, d) compacted granules or slug tablets are crushed and sieved through suitable sized sieve, e) granules in step (d) are mixed with the rest of disintegrant, f) lubricant is added and mixed, g) powder mixture in step (f) is filled into gelatin capsules or used for compression of core tablets.

11. The dry granulation, as claimed in claim 9, is used for manufacturing a pharmaceutical composition of anhydrous gemifloxacin mesylate comprises:
anhydrous gemifloxacin mesylate is in the range of from 10 % to 90 %, diluent is in the range of from 1 % to 50 %, binder is in the range of from 1 % to 10%, disintegrant is in the range of from 3 % to 15 %, lubricant is in the range of from 0.1 % to 2 % by weight of pharmaceutical dosage form, **more preferably** anhydrous gemifloxacin mesylate is in the range of from 15 % to 85 %, diluent is in the range of from 2 % to 25 %, binder is in the range of from 0.5 % to 3.5 %, disintegrant is in the range of from 4 % to 8 %, lubricant is in the range of from 0.3 % to 1.5 % by weight of pharmaceutical dosage form, **most preferably** anhydrous gemifloxacin mesylate is 79.80 %, diluent is 11.20%, binder is 2 %, disintegrant is 6 %, lubricant is 1% by weight of total pharmaceutical dosage form.

12. The dry granulation, as claimed in claim 11, is used for manufacturing a pharmaceutical composition of anhydrous gemifloxacin mesylate comprises ;
**a)** anhydrous gemifloxacin mesylate: diluent ratio is from 1:50 to 50:1 by weight, **more preferably** ratio is from 1:20 to 20:1, **most preferably** ratio is from1:10 to 10:1, **precisely** anhydrous gemifloxacin mesylate: diluent ratio is 7.12 **and/or, b)** anhydrous gemifloxacin mesylate: binder ratio is from 1:200 to 200:1 by weight, **more preferably** ratio is from 1:100 to 100:1, **most preferably** ratio is from1:50 to 50:1, **precisely** anhydrous gemifloxacin mesylate : binder ratio is 39.90 **and/or, c)** anhydrous gemifloxacin mesylate: disintegrant ratio is selected from 1:80 to 80:1 by weight, **more preferably** ratio is from 1:50 to 50:1, **most preferably** ratio is from1:20 to 20:1, **precisely** anhydrous gemifloxacin mesylate : disintegrant ratio is 13.30 **and/or, d)** anhydrous gemifloxacin mesylate: lubricant ratio is from 1:200 to 200:1 by weight, **more preferably** ratio is from 1:150 to 150:1, **most preferably** ratio is from 1:100 to 100:1, **precisely** anhydrous gemifloxacin mesylate : lubricant ratio is 79.80.
